# EUROPEAN PATENT SPECIFICATION

(11) **EP 1 879 568 B1**
(45) Date of publication and mention of the grant of the patent: **06.05.2009**
(21) Application number: 05764083.1
(22) Date of filing: 05.07.2005
(51) Int. Cl.: A61K 31/282, A61K 47/02

(54) **STABLE PHARMACEUTICAL COMPOSITIONS OF PLATINUM (II) ANTITUMOUR AGENTS**
STABILE PHARMAZEUTISCHE ZUSAMMENSETZUNGEN AUS ANTITUMORALEN PLATIN-(II)-MITTELN
PREPARATIONS PHARMACEUTIQUES STABLES D AGENTS ANTITUMORAUX CONTENANT DU PLATINE(II)

(30) Priority: 28.03.2005 IN KO02372005
(43) Date of publication of application: 23.01.2008
(73) Proprietor: Dabur Pharma Limited, Adjacent to Safdarjung Hospital New Delhi 110 029 (IN)
(72) Inventor: UPADHYAY, Satish Chandra, Sahibabad 201 010 (IN); PANANCHUKUNNATH, Manoj Kumar, Sahibabad 201 010 (IN); KUMAR, Dinesh, Sahibabad 201 010 (IN); SINGH, Ajeet Kumar, Sahibabad 201 010 (IN); MUKHERJEE, Rama, Sahibabad 201 010 (IN); BURMAN, Anand C., Sahibabad 201 010 (IN)
(74) Representative: Watson, Robert James
(86) International application number: PCT/IN2005/000228
(87) International publication number: WO 2006/103691

(56) References cited:
- US-A- 4 915 956
- US-A- 5 455 270
- US-A- 5 959 133
- DATABASE WPI Section Ch, Week 200508 Derwent Publications Ltd., London, GB; Class B05, AN 2005-075074 XP002347341 & WO 2004/112778 A1 (OTSUKA PHARM FACTORY INC) 29 December 2004 (2004-12-29)

## Description

### FIELD OF THE INVENTION

The present invention relates to stabilized pharmaceutical compositions of antitumor platinum (II) complexes and a process for preparation thereof.

### BACKGROUND OF THE INVENTION

Platinum (II) complexes have found wide acceptance for treatment of variety of tumors, especially Lung cancer, Lymphoma, Ovarian cancer, Testicular cancer, Bladder cancer, Urothelial cancer and Head/neck cancer in both humans and animals. Therapeutically and commercially important platinum complexes, which are currently in clinical practice, include cisplatin (Peyrone, M. Ann. Chemie Pharm. 1845, 51, 1-29), carboplatin (US 4,140,707), oxaliplatin (US 4,169,846) and miboplatin (US 4,822,892). Other platinum (II) complexes, which are at various stages of development, include lobaplatin and enloplatin.

In general, the above mentioned platinum (II) complexes are preferably administered to humans or animals affected with a tumor by an intravenous route i.e. an injectable. Such intravenous administration is essentially achieved through:
i) Reconstitution of a lyophilized or freeze dried powder of the requisite platinum (II) complex compound in an aqueous solvent, preferably water and administration of the solution thus obtained subsequent to dilution with dextrose or saline solutions; or
ii) Direct administration of a solution of the respective platinum (II) complex compound in an aqueous solvent, preferably water, by using recommended diluting solutions.

The former mode of administration is, however, associated with several disadvantages such as:
a) Double handling: To administer a lyophilized preparation, double handling of the drug is required. The lyophilized cake has to be first reconstituted and then administered;
b) Dissolution time of the cake: In some cases, the complete dissolution of the powder may require prolonged shaking because of solubilisation problems;
c) Health Hazard: Improper reconstitution of a lyophilized powder sometimes result in the formation of air-borne droplets ("blow-back"), which, in the case of a potent antitumor agent such as platinum complexes may be a health hazard to the personnel making up the solution for injection;
d) Improper dose: There is always a problem in reconstituting a lyophilized powder in that an inappropriate quantity of diluents may be used because of a different vial size. This could result in a improper dose being administered to a patient; and
e) Cost of manufacture: The manufacture of a lyophilized formulation is quite costly, since it not only requires capital investment for installation of a lyophiliser, but also its maintenance;
   even though, the stability of the reconstituted solution is not a major issue, since such solutions need to be administered immediately or within a prescribed time, generally not exceeding 8 hrs.

A preformed solution of platinum (II) complex compounds in aqueous solvents, generally referred to as "ready-to-use" solution, has found wide utility in comparison to a solution reconstituted from a lyophilizate as it overcomes the limitations associated with a lyophilized composition. However, storage stability of such ready-to-use solutions are a major concern, which has vexed researchers and manufacturers of such solutions since long.

There is a welter of literature, both academic and patents, which describe the studies directed towards not only understanding the mechanistic and/or kinetic pathway of the degradation of platinum (II) complex solutions but also in stabilization of such solutions. To name a few, stabilization of platinum (II) complex solutions have been reported to be achieved through:
a) Maintenance and/or adjustment of pH of a cisplatin solution to 3.5 - 5.0 and excluding any dissolved oxygen in the said solution as disclosed by Alam et. al. in US 4,915,956.
   The patent claims that for obtaining optimum stability, the level of dissolved oxygen in the solution should be less than 2 ppm.
b) Addition of aqueous polyethylene glycol, methoxy polyethylene glycol and a source of chloride ions in a cisplatin solution as disclosed by Kaplan et. al. in US 4,451,447.
c) Adjustment of the pH of a cisplatin solution to 2.0 - 3.0 with acids, such as hydrochloric acid, as disclosed by Granatek et. al. in US 4,310,515.
d) Addition of preservatives, like benzyl alcohol and mannitol, to a carboplatin solution as disclosed by Levius et. al. in EP 0,334,551.
e) Adjustment of the pH to 2.0 - 6.5 of a carboplatin solution by inorganic buffers as disclosed by Nijkerk et. al. in US 5,104,896.
f) Addition of a requisite amount of 1,1-cyclobutane dicarboxylic acid to a carboplatin solution and adjusting the pH of the solution to 4.0 - 8.0 as disclosed by Kaplan et. al. in US 5,455,270 or adjusting the pH to 2.0 - 7.0 as disclosed by Kiss et. al. in EP 0,743,854.
g) Utilization of carboplatin having less than 0.1% by weight of 1,1-cyclobutane dicarboxylic acid for preparation of a carboplatin solution as disclosed by Kysilka et. al. in US 6,589,988.
h) Addition / utilization of oxalic acid or its salt as a buffering agent in an oxaliplatin solution as disclosed by Anderson et. al. in US 6,306,902.
i) Addition / utilization of lactic acid or malonic acid or their salts as a buffering agent in an oxaliplatin solution as disclosed by Lauria et. al. in US 6,476,068 or in published US Application No. 2003/0,109,515 respectively.
j) Addition / utilization of a carbohydrate or an inorganic and/or organic acid in an oxaliplatin solution as disclosed by Schridde et. al in published EP Application Nos. 1,466,599 Al and 1,466,600 A1.
k) Utilization of oxaliplatin, having not more than 0.08% by weight of oxalic acid, for preparation of an oxaliplatin solution as disclosed by Ibrahim et. al. in published US Application No. 2004/0,186,172.
l) Utilization of a platinum (II) complex compound prepared under conditions which strictly exclude oxygen and utilization of the said platinum (II) compound thus obtained for preparation of the respective 'ready-to-use solution' as disclosed by Ohnishi et al in US 5,959,133.

From the abovementioned disclosures, it would be apparent that most, if not all the methods for stabilization of platinum (II) complex compound solution involve:
i) Utilization of additives such as physiologically acceptable acids, physiologically acceptable buffers, carbohydrates, preservatives, antioxidants, a stabilizing amount of corresponding dicarboxylic acids etc.;
ii) Adjustment and /or maintenance of the pH of platinum (II) complex compound solutions;
iii) Utilization of a platinum (II) complex active pharmaceutical ingredient (API) having very low content, generally below 0.1% by weight, of the corresponding dicarboxylic acid; and
iv) Exclusion of all dissolved oxygen from the solutions or through utilization of platinum (II) complex APIs prepared under conditions, which strictly exclude oxygen from the entire manufacturing process.

However, the abovementioned methods suffer from one or more of the following limitations, which render such methods either not economical, convenient or commercially not particularly viable. The limitations are
i) Selection of an appropriate additive for stabilization. For instance, there is neither any general guideline nor can an inference be drawn that stabilization achieved in an oxaliplatin solution through addition of either oxalic acid, lactic acid, malonic acid, carbohydrates or organic and/or inorganic acid would give similar or substantially similar stabilization to a cisplatin, carboplatin or miboplatin solution through utilization of the said additive.
ii) Similarly, there is neither any general guideline nor can an inference be drawn that addition of a stabilizing amount of cyclobutane dicarboxylic acid to a carboplatin solution would result in similar or substantially similar stabilization of an oxaliplatin solution or for that matter a cisplatin or miboplatin solution by addition of the respective dicarboxylic acid solution.
iii) The disclosures in US 4,915,956 and in US 5,959,133 amply demonstrate that oxygen present in the system either in the dissolved state in the solution or arising and/or carried forward from the active pharmaceutical ingredient utilized for its preparation are detrimental to the stability of such solutions by virtue of the fact that oxygen present in the system actually facilitates the formation of over oxidation products which not only leads to lower potency but also imparts coloration to such solutions.

Further, it might be mentioned that for manufacture of an active pharmaceutical ingredient having very low content of the respective dicarboxylic acid more often than not, recourse to tedious and costly purification techniques are necessary, which needless to mention, would increase the cost of manufacturing.

Furthermore, for exclusion of oxygen from either the active pharmaceutical ingredient or the finished dosage forms recourse to control of systems for effecting complete or near complete exclusion of the said gas i.e. oxygen is necessary, which would increase not only the cost of manufacturing but also the hazards and operability of the methods.

In addition, even though, the disclosure contained in US 5,455,270 does mention use of oxygen or air i.e. a gaseous mixture of about 78% nitrogen and 21% oxygen in preparation of carboplatin solution, however from the teachings of the said patent it would be apparent that in preparation of such solution the gas is invariably bubbled into a solution of carboplatin in water prior to sterilization and filling the solution in vials or alternatively filling a sterilized solution of carboplatin in water in suitable vials and blanketing the free headspace of the said vial with air.

However it should be noted that as per the teachings of the US 5,455,270 bubbling of gas or blanketing the headspace with air is an optional embodiment of the invention recited therein, the eventual invention, however, comprises stabilization of a carboplatin solution through addition of requisite amount of 1,1-cyclobutane dicarboxylic acid (CBDCA).

Further, the disclosure of US 5,455,270 is not enabling enough in that it does not specify the amount of oxygen to be bubbled. Moreover, patent claims that whenever a purging technique is employed, it is preferred that the liquid in the container fill no more than 50% of its volume (half full), i.e. the unfilled air oxygen volume or headspace be more than 50% of the total volume of the container.

It might be further mentioned that Health Authorities all over the world are very concerned about the level of degradation product and impurities present in a drug substance or a drug product. As a consequence, regulatory approval norms today are very stringent about the level of impurities present in a drug substance or a drug product. In view of this, it is rather intriguing how a platinum (II) complex compound solution containing more often than not amounts of additives above the limits prescribed by regulatory authorities could comply with pharmacopeial specifications, even though such solutions may be stable.

From the foregoing, it would be apparent that there is no universal method or system for stabilization of a platinum (II) complex compound solution, which is simple, convenient, economical and is not dependent on the vagaries of critical parameters like pH, amount of additives specially requisite dicarboxylic acid, amount of oxygen present, quality of active pharmaceutical ingredient etc.

A need, therefore, exists for a pharmaceutical composition of platinum (II) complex compounds which is universal, simple, convenient, and is not dependent on the vagaries of critical parameters like pH, amount of additives specially requisite dicarboxylic acid, amount of oxygen present, quality of active pharmaceutical ingredient etc.

The present invention is a step forward in this direction and overcomes most, if not all the limitations of the prior art methods in providing a novel and simple method for stabilization of platinum (II) complex compound solutions.

### OBJECTS OF THE INVENTION

An object of the present invention is to provide a pharmaceutical composition of platinum (II) complex compounds, which are stable on storage for pharmaceutically acceptable duration of time.

Another object of the present invention is to provide a pharmaceutical composition of platinum (II) complex compounds, which are stable and undergo less degradation.

Yet another object of the present invention is to provide a process for preparation of a stable pharmaceutical composition of platinum (II) complex compounds, which is simple, convenient and economical.

A further object of the present invention is to provide a use of such stable pharmaceutical compositions of platinum (II) complex compounds for the manufacture of a medicament for the treatment of a human or an animal cancerous disease.

### SUMMARY OF THE INVENTION

Thus the present invention relates to a stable pharmaceutical composition comprising a platinum (II) complex compound in an oxygen enriched aqueous solvent wherein the aqueous solvent has the dissolved oxygen concentration in the range from 20 ppm to 40 ppm and the said composition is being filled in a container without having blanket gas in head space

According to another aspect the invention relates to a process for preparing a pharmaceutical composition comprising a platinum (II) complex compound in an oxygen enriched aqueous solvent comprising the steps of:
(i) Bubbling of a gas into an aqueous solvent for a suitable period of time to enrich the dissolved oxygen content of the aqueous solvent;
(ii) Addition of platinum (11) complex compound to the oxygen enriched aqueous solvent of step (i);
(iii) Mixing or agitating the mixture of step (ii) to obtain a clear solution;
(iv) Sterilizing the solution of step (iii);
(v) Filling the sterilized, clear solution of step (iv) into suitable containers
   wherein the head space is not filled with a gas;
(vi) Sealing the containers as prepared in step (v).

According to a further aspect there is also provided a device for preparing a pharmaceutical composition as claimed in claim 1, wherein the process according to claim 8 is carried out in an assembly having sparger surface pore size from 0.2 µm to 7874 µm (0.31 inch).

According to a still further aspect there is also provided a Use of a pharmaceutical composition as claimed in claim 1 for the manufacture of medicament for the treatment of a human or an animal cancerous disease.

In their efforts to prepare a ready-to-use solution of platinum (II) complex compounds, in particular carboplatin, the present inventors have found to their surprise that such a solution could be made to stand for a pharmaceutically acceptable duration of time without significant drop in potency as well as minimizing the formation of degradation products through a simple unit operation of enriching the oxygen content of the solution.

Further, the present inventors have found that other than enriching oxygen content of the solution, no additive needs to be added, no pH adjustment is required and no particular significance need to be given for active pharmaceutical ingredient specification, i.e. the dicarboxylic acid content, for stabilization of the solution. In particular, it has been found that a stable solution of carboplatin in water can be obtained by simple dissolution of carboplatin in an oxygen-enriched water, involving neither addition of any stabilization agent nor adjustment of pH with a pharmaceutically acceptable pH modifier.

Furthermore, it has been found that the acceptable stability of the pharmaceutical composition of the present invention could be achieved through utilization of water, which is at least 50% saturated with oxygen, with maximum stability being observed when the water used for injection is saturated or near saturated with oxygen.

The method for preparation of such stable solutions is simple and is achieved through bubbling of oxygen gas into an aqueous solvent for a suitable period of time, followed by addition of platinum (II) complex compound to obtain solutions, which can be made into a sterile form for human or animal consumption by conventional methods.

Further, it has been found that instead of oxygen when any other gas like nitrogen is bubbled into an aqueous solvent, which is then utilized for the preparation of a solution of platinum (II) complex compounds, the stability profile of such solution is far inferior to those wherein no gas is bubbled. This is not surprising, since bubbling of nitrogen into an aqueous solvent actually drives out any dissolved oxygen thereby providing an oxygen depleted solution, which in turn leads to the inferior stability. The stabilizing effect of an oxygen enriched composition prepared as per the method of the present invention over that of a composition not enriched or depleted of oxygen could be best understood by a comparative stability profile of the two compositions as summarized in Table - I. Also for direct comparison the stability profile of a solution prepared by bubbling of nitrogen is summarized in Table - I.

**Table - I**

| *Comparison of Stability of Carboplatin Solutions enriched with oxygen with those not enriched with oxygen.* | | | | |
|---|---|---|---|---|
| Solution of carboplatin enriched with | Storage conditions | Solution Description | Carboplatin Assay (mg/ml) | % Fall in assay of Carboplatin |
| Nitrogen | Initial | Clear colourless solution | 10.20 | -- |
| | 20D/50°C | Black solution | NE | NE |
| None | Initial | Clear colourless solution | 10.53 | -- |
| | 15 D / 60°C | yellow coloured solution | 9.72 | 6.1 |
| | 30 D / 50°C | Light brown coloured solution | 10.13 | 4.0 |
| Oxygen | Initial | Clear colourless solution | 10.17 | -- |
| | 15 D / 60°C | Very light yellow coloured solution | 9.57 | 6.0 |
| | 30 D / 50°C | Faint yellow coloured solution | 9.95 | 2.2 |

| | | | | |
|---|---|---|---|---|
| D- days, NE- not evaluated, CBDCA- cyclobutane dicarboxylic acid | | | | |

Furthermore, the present inventors have also found that the acceptable stability of the pharmaceutical composition of the present invention is not determined by the volume of the solution filled in the container or to put in other words the stability is not dependent on the free available headspace of the container. It has been found that a container filled with an oxygen enriched carboplatin solution in an aqueous solvent less than or more than 50% of the volume of the container exhibit essentially similar stability or degradation profile.

Thus, in accordance with the above:

In one aspect, the present invention provides a stable pharmaceutical composition comprising a platinum (II) complex compound in an oxygen enriched aqueous solvent wherein the aqueous solvent has the dissolved oxygen concentration in the range from 20 ppm to 40 ppm and the said composition is being filled in a container without having blanket gas in head space.

In another aspect of the present invention, the stable pharmaceutical compositions comprise a solution of platinum (II) complex compounds in an aqueous solvent having a pH from about 5.0 to 7.0.

In yet another aspect of the present invention, the stable pharmaceutical compositions comprise a solution of platinum (II) complex compounds in an aqueous solvent having a pH from about 5.0 to 7.0 contained in a suitable container wherein the free headspace of the container is not blanketed with a gas.

In still another aspect of the present invention, the stable pharmaceutical compositions comprise a solution of platinum (II) complex compounds in an aqueous solvent having a pH from about 5.0 to 7.0 contained in a suitable container wherein the volume of the platinum (II) solutions is less than 50% of the volume of the container.

In a further aspect of the present invention, the stable pharmaceutical compositions comprise a solution of platinum (II) complex compounds in an aqueous solvent having a pH from about 5.0 to 7.0 contained in a suitable container wherein the volume of the platinum (II) solutions is more than 50% of the volume of the container.

In yet further aspect, the present invention provides a process for preparation of stable pharmaceutical composition of platinum (II) complex compounds in an aqueous solvent having a pH from about 5.0 to 7.0 comprising the steps of:
i) Bubbling of oxygen gas into an aqueous solvent for a suitable period of time;
ii) Addition of platinum (II) complex compound to the solution of step (i);
iii) Mixing or agitating the mixture of step (ii) to obtain a clear solution;
iv) Sterilizing the solution of step (iii);
v) Filling the sterilized, clear solution of step (iv) into suitable containers; and
vi) Sealing the containers as prepared in step (v).

In another aspect, the present invention provides a process as in claim 8 for preparation of stable pharmaceutical composition of platinum (II) complex compounds in an aqueous solvent having a pH from about 5.0 to 7.0 wherein the entire process is carried out in assembly as depicted in Fig - 1.

In yet further aspect of the present invention, the stable pharmaceutical compositions comprise a solution of carboplatin in an aqueous solvent having a pH from about 5.0 to 7.0 contained in a suitable container wherein the volume of the carboplatin solutions is less than 50% of the volume of the container.

In another aspect of the present invention, the stable pharmaceutical compositions comprise a solution of carboplatin in an aqueous solvent having a pH from about 5.0 to 7.0 contained in a suitable container wherein the volume of the carboplatin solutions is more than 50% of the volume of the container.

In yet another aspect, the present invention provides a process for preparation of stable pharmaceutical composition of carboplatin in an aqueous solvent having a pH from about 5.0 to 7.0 comprising the steps of:
i) Bubbling of oxygen gas into an aqueous solvent for a suitable period of time;
ii) Addition of carboplatin to the solution of step (i);
iii) Mixing or agitating the mixture of step (ii) to obtain a clear solution;
iv) Sterilizing the solution of step (iii);
v) Filling the sterilized, clear solution of step (iv) into suitable containers and
vi) Sealing the containers as prepared in step (v).

In still another aspect of the present invention, the entire process for preparation of the stable pharmaceutical composition of carboplatin in an aqueous solvent having a pH from about 5.0 to 7.0 is carried out in assembly as depicted in Fin - 1.

### BRIEF DESCRIPTION OF THE FIGURES

Fig - 1 depicts a schematic description of the assembly used to carry out the process for preparation of a stable aqueous pharmaceutical composition of platinum (II) complex compounds. The assembly consists of a sparger unit, stirrer and dissolved oxygen meter probe to monitor the level of dissolved oxygen. Sparger unit consists of an oxygen cylinder, flow regulator, pressure regulator, filter, tubings and sparger.

### DETAILED DESCRIPTION OF THE INVENTION

The oxygen enriched aqueous solvent is obtained by purging or bubbling of a gas into an aqueous solvent for a suitable period of time, to which is added the platinum (II) complex compound and mixed or agitated to obtain a clear solution. Preferably, the solution has a pH of from about 5.0 to 7.0. The platinum (II) complex compound is selected from cisplatin, carboplatin, oxaliplatin, lobaplatin, enloplatin and miboplatin.

The aqueous solvent is selected from the group consisting of water, aqueous polyalkylene glycols containing C₁₋₆ alkyl groups, aqueous carbohydrate solutions and mixtures thereof. The most preferred aqueous solvent is water.

The final water content in the solution of platinum (II) complex compound ranges from 0.01 to 99.99%. The gas used in the process is selected from oxygen, oxygen allotropes and combinations thereof. The purging of the gas into the aqueous solvent is carried out for a period from about 30 minutes to about 150 minutes and preferably from about 90 minutes to about 120 minutes.

After the enrichment of oxygen into the aqueous solvent, the dissolved oxygen concentration is in the range from about 20 ppm to about 40 ppm. The preferred dissolved oxygen concentration is 35 ppm.

As mentioned hereinbefore, the pharmaceutical compositions of the present invention comprising of an aqueous solution of a platinum (II) complex compounds is achieved by simple dissolution of the platinum (II) compounds in an oxygenated aqueous solvent.

As used herein, the term "platinum (II) complex compounds" refers to platinum compounds having 2+ oxidation state in aqueous solution, which dictate the square planar sterochemical arrangement of the ligand. Examples of such platinum (II) complex compounds are, but not limited to, cisplatin, carboplatin, oxaliplatin, myboplatin, lobaplatin, enloplatin etc.

As used herein the term "aqueous solvent" refers to water containing solvents. Water for injection is preferred solvent. Mixtures of water and one or more auxiliary carriers e.g. polyalkylene glycols and sugar solutions could be employed. Typically, the final water content in the solution of the invention could range from 0.1 to 99.9% with auxiliary carriers. Suitable glycols include polyalkylene glycols having molecular weight of about 300 to about 900 and bearing C₁₋₆ alkyl groups. Accordingly, polyether polyols such as polyethylene glycol, polypropylene glycol, polybutylene glycol and the like and the mixtures thereof can be used. "Sugar solution" includes solutions of pharmaceutically acceptable dextrose, sucrose, mannose or other sugars which function as isotonicity adjusting agents.

As used herein the term "oxygenated or oxygen enriched" solvent means an aqueous solvent containing dissolved oxygen obtained by means of aerating the aqueous solvent with oxygen gas. The aeration of oxygen in the aqueous solvent could be attained by bubbling the oxygen gas or any allotrope of oxygen or combinations thereof.

The term "bubbling" means that sparging or otherwise passing the oxygen gas through the aqueous solvent under pressure to increase the dissolved oxygen level of the aqueous solvent. The preferred dissolved oxygen level is from about 20 ppm to 40 ppm. The solution having dissolved oxygen level of around 35 ppm i.e. saturated or nearly saturated solutions are preferred.

The amount of carboplatin used in the formulation according to present invention vary from about 1.0 mg to 22.0 mg/ml. The amount, which is present, is not critical and may be adjusted in accordance with the individual needs and preferences. Typically, the concentration of carboplatin will be about 10.0 mg/ml.

The pharmaceutical composition of the present invention can be prepared in an assembly as depicted in Fig - 1. The process comprises of the following steps:
i) Enriching the dissolved oxygen content of an aqueous solvent
ii) Addition of platinum (II) complex compound to the solution of step (i);
iii) Mixing or agitating the mixture of step (ii) to obtain a clear solution;
iv) Sterilizing the solution of step (iii);
v) Filling the sterilized, clear solution of step (iv) into suitable containers; and
vi) Sealing the containers as prepared in step (v).

To enrich the aqueous solvent with oxygen gas, the oxygen gas is introduced into the aqueous solvent by means of a sparger. "Sparging" is a means of bubbling of an oxygen gas through a solution under suitable pressure to get the desired level of dissolved oxygen in aqueous solvent. The sparger can be constructed from a material selected from the group consisting of carbon steel and low-alloy steels or elastomeric material. The tube sparger preferably consists of a perforated elastomer tube, or perforated / sintered steel tube. The volumetric flow rate of the oxygen gas and/or an allotrope containing gas added via the tube sparger is 0.01 to 0.4 m³ (STP)/h per m³. The gas is introduced into the carrier through several, usually thousands of tiny pores, creating small fine bubbles. The sparger surface pore size can vary from 0.2 µm to the 0.31 inch. Pore size is the most important parameter to be considered in the design of the gas sparging system. This particular sparger pore size range allows to attain a sufficient sparge pressure and gas flow enhancing the mass transfer. The period for which the aqueous solvent is purged is generally between about 30 minutes and about 150 minutes. Periods of about 1.5 hrs to about 2.0 hrs are preferred. The duration of purging is not critical, however it is generally desirable to sparge in aqueous solvent to achieve half saturated or complete or near complete saturated solution with the oxygen gas i.e. having dissolved oxygen content from about 20 ppm to about 40 ppm. The optimum stability is achieved with nearly saturated solution viz. having dissolved oxygen content of about 35 ppm. Under the above mentioned conditions, the desired saturation level is achieved by bubbling the gas for around 90 minutes into an aqueous solvent.

Suitable packaging for the platinum (II) complex compound solutions may be the approved containers for parenteral use, such as plastic and glass containers, ready-to-use syringes and the like. Preferably the container is a sealed glass container, e.g vial or an ampoule. A hermetically sealed glass vial is the preferred container.

The pharmaceutical compositions thus prepared exhibit excellent storage stability as would be evident from the examples given hereinbelow .

The pharmaceutical compositions are highly effective for treatment in both human and animal hosts, of tumors, such as sarcomas, carcinomas of prostate, lung, breast, head and neck, bladder, urothelium, thyroid, ovary, testes, etc., lymphomas including Hodgkin and no-Hodgkin, neuroblastoma, leukaemias including acute lymphoblastic leukemia and acute myeloblastic leukemia, Wilm's tumor, melanoma, myeloma etc.

The pharmaceutical compositions can be administered by rapid intravenous injection or infusion. For e.g. a composition containing carboplatin can be administered by an intravenous infusion of 175 to 600 mg/m² given in a single infusion on day 1 every 4 weeks.

### Example - 1

A carboplatin solution having a concentration of around 10 mg/ml was prepared as follows: First, the oxygen enriched aqueous solvent was prepared by purging (bubbling) oxygen gas into water for 1.5 hour with the help of assembly shown in Fig - 1. Then, the requisite amount of carboplatin was added to the oxygen enriched water thus obtained to get a solution of carboplatin having concentration of 10 mg/ml. Sparging and agitation was continued throughout the addition operation and until the carboplatin was observed to be visibly dissolved. The clear solution was then passed through a sterile filter under positive pressure and then aseptically filled into vials. The carboplatin solution filled vials were stored at various storage conditions. The pH of each solution was measured and the content of carboplatin and cyclobutane dicarboxylic acid were determined by HPLC. This was done with the solution as originally prepared and after various storage conditions. The results of all these studies are summarized in Table - II.

**Table - II**

| *Carboplatin Physical and Chemical stability at 25°C, 30°C, 40°C and 60°C.* | | | |
|---|---|---|---|
| Storage Conditions | Description | pH | Carboplatin assay % |
| Initial | Clear colorless solution | 5.80 | 103.9 |
| 2W / 60°C | Light yellow coloured solution | 5.78 | 96.9 |
| 1M / 50°C | Clear colorless solution | 5.70 | 93.5 |
| 1M / 40°C | Clear colorless solution | 5.90 | 95.1 |
| 2M / 50°C | Yellow colored solution | 5.70 | 95.2 |
| 2M / 40°C | Clear colorless solution | 5.86 | 98.4 |
| 2M / 30°C | Clear colorless solution | 6.00 | 101.9 |
| 3M / 25°C | Clear colorless solution | 5.80 | 97.5 |
| 3M / 30°C | Clear colorless solution | 5.78 | 97.1 |

### Example -2

To determine the effect of oxygen gas on stability of platinum (II) complex compound solutions, carboplatin solutions enriched with oxygen, and not enriched with oxygen were prepared. The oxygen enriched solution was prepared in a manner similar to that described in Example - 1. The carboplatin solutions not enriched with oxygen were prepared as follows: Nitrogen gas was purged into water for 1.5 hour with the help of assembly as shown in Fig - 1. Then the requisite amount of carboplatin was added to the nitrogen purged solution to get the final concentration of 10 mg/ml. Sparging and agitation was continued throughout the addition operation and until the carboplatin was observed to be visibly dissolved. The clear solution was sterilized and filled into vials in a manner similar to that described in Example -1.

Similarly, carboplatin solution was prepared without purging any gas into the water. All the solutions were stored at various storage conditions and the stability of these solutions were determined. The results are given in Table -I.

**Table - I:**

| *Comparison of Stability of Carboplatin Solutions enriched with oxygen with those not enriched with oxygen.* | | | | |
|---|---|---|---|---|
| Solution of carboplatin enriched with | Storage conditions | Solution Description | Carboplatin Assay (mg/ml) | % Fall in assay of Carboplatin |
| Nitrogen | Initial | Clear colourless solution | 10.20 | ---- |
| | 20 D/ 50°C | Black solution | NE | NE |
| None | Initial | Clear colourless solution | 10.53 | ---- |
| | 15 D / 60°C | Yellow coloured solution | 9.72 | 6.1 |
| | 30 D / 50°C | Light brown coloured solution | 10.13- | 4.0 |
| Oxygen | Initial | Clear colourless solution | 10.17 | ---- |
| | 15 D / 60°C | Very light yellow coloured solution | 9.57 | 6.0 |
| | 30 D / 50°C | Faint yellow solution | 9.95 | 2.2 |

| | | | | |
|---|---|---|---|---|
| D- days, NE- not evaluated, CBDCA- cyclobutane dicarboxylic acid | | | | |

### Example -3

The effect of vial fill volume i.e. headspace on the stability of aqueous solution of carboplatin was also determined at 50°C and 60°C. Carboplatin solutions having concentration 10 mg/ml were prepared by using oxygen saturated water in a manner similar to that of Example - 1. After preparation, solutions were sterilized by filtration and filled aseptically into glass vials with headspace variation of more than and less than 50% of the volume of the container. Samples were assayed after 15 and 30 days. The stability data of these solutions is given in Table - III and IV.

**Table -III**

| *Effect of Headspace and Oxygen sparging on carboplatin solution stability* | | | | | |
|---|---|---|---|---|---|
| Headspace | Condition | Description | Assay | %Fall in assay | Color ratio (Initial absorbance to exposed sample absorbance) |
| ≤ 50% | Initial | Clear colorless solution free from any visible particles | 10.17 | - | 0.002 |
| | 1M / 40°C / 75%RH | Clear colorless solution free from any visible particles | 9.95 | 2.2 | 0.006 |
| ≥ 50% | Initial | Clear colorless solution free from any visible particles | 10.00 | - | <0.001 |
| | 1M 140°C / 75%RH | Clear colorless solution free from any visible particles | 9.88 | 1.2 | 0.011 |

**Table - IV**

| *Effect of Headspace and Oxygen sparging on carboplatin solution stability.* | | | | | |
|---|---|---|---|---|---|
| | Condition | Description | Assay (mg/ml) | %Fall in assay | Color ratio (Initial absorbance to exposed sample absorbance) |
| Headspace ~10% | Initial | Clear colorless solution | 10.17 | - | - |
| | 15 D/ 60°C | Clear faintly yellow solution | 9.57 | 6.0 | 10 |
| | 30 D/ 50°C | Clear faintly yellow solution | 9.95 | 2.2 | 5.5 |
| Headspace ~45% | Initial | Clear colorless solution | 10.39 | - | - |
| | 15 D/ 60°C | Clear faintly yellow solution | 9.74 | 6.5 | 9.5 |
| | 30 D/ 50°C | Clear faintly yellow solution | 9.99 | 4.0 | 5.2 |

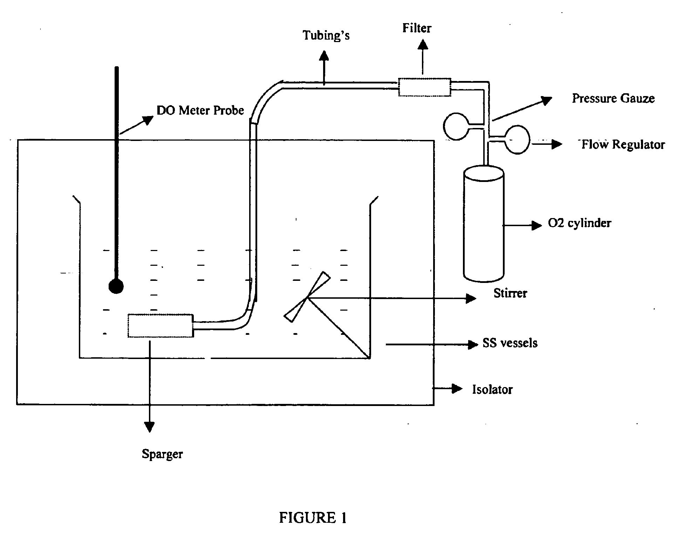

## Claims

1. A stable pharmaceutical composition comprising a platinum (II) complex compound in an oxygen enriched aqueous solvent wherein the aqueous solvent has the dissolved oxygen concentration in the range from 20 ppm to 40 ppm and the said composition is being filled in a container without having blanket gas in head space.

2. A pharmaceutical composition as claimed in claim 1, wherein the oxygen enriched aqueous solvent has the dissolved oxygen concentration of about 35 ppm.

3. A pharmaceutical composition as claimed in claim 1, wherein the said composition has a pH of from 5.0 to 7.0.

4. A pharmaceutical composition as claimed in claim 1, wherein the platinum (II) complex compound is selected from cisplatin, carboplatin, oxaliplatin, lobaplatin, enloplatin and miboplatin.

5. A pharmaceutical composition as claimed in claim 1, wherein the aqueous solvent is selected from the group consisting of water, aqueous polyalkylene glycols containing C₁₋₆ alkyl groups, aqueous carbohydrate solutions and mixtures thereof.

6. A pharmaceutical composition as claimed in claim 5, wherein the aqueous solvent is water.

7. A pharmaceutical composition as claimed in claim 6, wherein the final water content in the solution of platinum (II) complex compound ranges from 0.01 to 99.99%.

8. A process for preparing a pharmaceutical composition comprising a platinum (II) complex compound in an oxygen enriched aqueous solvent comprising the steps of:
(i) Bubbling of a gas into an aqueous solvent for a suitable period of time to enrich the dissolved oxygen content of the aqueous solvent;
(ii) Addition of platinum (11) complex compound to the oxygen enriched aqueous solvent of step (i);
(iii) Mixing or agitating the mixture of step (ii) to obtain a clear solution;
(iv) Sterilizing the solution of step (iii);
(v) Filling the sterilized, clear solution of step (iv) into suitable containers
wherein the head space is not filled with a gas;
(vi) Sealing the containers as prepared in step (v).

9. A process as claimed in claim 8 for preparing a pharmaceutical composition
wherein said gas is selected from oxygen, oxygen allotropes and combinations thereof.

10. A process as claimed in claim 9, for preparing a pharmaceutical composition
wherein said bubbling of said gas into said aqueous solvent is carried out for a period from 30 minutes to 150 minutes.

11. A process as claimed in claim 9, for preparing a pharmaceutical composition
wherein said bubbling of said gas into said aqueous solvent is carried out preferably for a period from 90 minutes to 120 minutes.

12. A device for preparing a pharmaceutical composition as claimed in claim 1, wherein the process according to claim 8 is carried out in an assembly having sparger surface pore size from 0.2 µm to 7874 µm (0.31 inch).

13. Use of a pharmaceutical composition as claimed in claim 1 for the manufacture of medicament for the treatment of a human or an animal cancerous disease.

## Patentansprüche

1. Stabile pharmazeutische Zusammensetzung, umfassend eine Platin(II)-Komplexverbindung in einem sauerstoffangereicherten wässrigen Lösungsmittel, worin das wässrige Lösungsmittel eine Konzentration an gelöstem Sauerstoff im Bereich von 20 ppm bis 40 ppm aufweist und die Zusammensetzung ohne Schutzgas im Kopfraum in einen Behälter gefüllt wird.

2. Pharmazeutische Zusammensetzung nach Anspruch 1, worin das sauerstoffangereicherte wässrige Lösungsmittel eine Konzentration an gelöstem Sauerstoff von etwa 35 ppm aufweist.

3. Pharmazeutische Zusammensetzung nach Anspruch 1, worin die Zusammensetzung einen pH von 5,0 bis 7,0 hat.

4. Pharmazeutische Zusammensetzung nach Anspruch 1, worin die Platin(II)-Komplexverbindung aus Cisplatin, Carboplatin, Oxaliplatin, Lobaplatin, Enloplatin und Miboplatin ausgewählt ist.

5. Pharmazeutische Zusammensetzung nach Anspruch 1, worin das wässrige Lösungsmittel aus der aus Wasser, wässrigen Polyalkylenglykolen, die C₁₋₆-Alkylgruppen enthalten, wässrigen Kohlenwasserstofflösungen und Gemischen davon bestehenden Gruppe ausgewählt ist.

6. Pharmazeutische Zusammensetzung nach Anspruch 5, worin das wässrige Lösungsmittel Wasser ist.

7. Pharmazeutische Zusammensetzung nach Anspruch 6, worin der Endwassergehalt in der Lösung der Platin(II)-Komplexverbindung in einem Bereich von 0,01 bis 99,99 % liegt.

8. Verfahren zur Herstellung einer pharmazeutischen Zusammensetzung, umfassend eine Platin(II)-Komplexverbindung in einem sauerstoffangereicherten wässrigen Lösungsmittel, umfassend die nachstehenden Schritte:
(i) das Durchperlen eines Gases in ein wässriges Lösungsmittel über eine geeignete Zeitdauer, um den Gehalt an gelöstem Sauerstoff im wässrigen Lösungsmittel anzureichern;
(ii) das Zusetzen einer Platin(II)-Komplexverbindung zu dem sauerstoffangereicherten wässrigen Lösungsmittel aus Schritt (i);
(iii) das Vermischen oder Rühren des Gemischs aus Schritt (ii), um eine klare Lösung zu erhalten;
(iv) das Sterilisieren der Lösung aus Schritt (iii);
(v) das Einfüllen der sterilisierten, klaren Lösung aus Schritt (iv) in geeignete Behälter, in denen der Kopfraum nicht mit einem Gas befüllt ist;
(vi) das Abdichten der wie in Schritt (v) vorbereiteten Behälter.

9. Verfahren nach Anspruch 8 zur Herstellung einer pharmazeutischen Zusammensetzung, worin das Gas aus Sauerstoff, Sauerstoffallotropen oder Kombinationen davon ausgewählt ist.

10. Verfahren nach Anspruch 9 zur Herstellung einer pharmazeutischen Zusammensetzung, worin das Durchperlen des Gases in das wässrige Lösungsmittel 30 min bis etwa 150 min lang durchgeführt wird.

11. Verfahren nach Anspruch 9 zur Herstellung einer pharmazeutischen Zusammensetzung, worin das Durchperlen des Gases in das wässrige Lösungsmittel vorzugsweise 90 min bis 120 min lang durchgeführt wird.

12. Vorrichtung zur Herstellung einer pharmazeutischen Zusammensetzung nach Anspruch 1, worin das Verfahren nach Anspruch 8 in einer Anordnung mit einer Einblasrohr-Oberflächenporengröße von 0,2 µm bis 7.874 µm (0,31 Zoll) durchgeführt wird.

13. Verwendung einer pharmazeutischen Zusammensetzung nach Anspruch 1 zur Herstellung eines Medikaments zur Behandlung einer Krebserkrankung in einem Menschen oder einem Tier.

## Revendications

1. Composition pharmaceutique stable comprenant un composé complexe du platine (II) dans un solvant aqueux enrichi d'oxygène, dans laquelle le solvant aqueux possède la concentration d'oxygène dissous dans la plage de 20 ppm à 40 ppm, et ladite composition est introduite dans un contenant sans avoir du gaz de couverture dans un espace de tête.

2. Composition pharmaceutique selon la revendication 1, dans laquelle le solvant aqueux enrichi d'oxygène possède la concentration d'oxygène dissous d'environ 35 ppm.

3. Composition pharmaceutique selon la revendication 1, dans laquelle ladite composition possède un pH de 5,0 à 7,0.

4. Composition pharmaceutique selon la revendication 1, dans laquelle le composé complexe du platine (II) est sélectionné parmi cisplatine, carboplatine, oxaliplatine, lobaplatine, enloplatine et miboplatine.

5. Composition pharmaceutique selon la revendication 1, dans laquelle le solvant aqueux est sélectionné dans le groupe consistant en eau, polyalkylène glycols aqueux contenant des groupes alkyle C₁₋₆, des solutions aqueuses d'hydrate de carbone et leurs mélanges.

6. Composition pharmaceutique selon la revendication 5, dans laquelle le solvant aqueux est de l'eau.

7. Composition pharmaceutique selon la revendication 6, dans laquelle la teneur finale en eau dans la solution du composé complexe du platine (II) s'étend de 0,01 à 99,99%.

8. Procédé de préparation d'une composition pharmaceutique comprenant un composé complexe du platine (II) dans un solvant aqueux enrichi d'oxygène comprenant les étapes de:
(i) bullage d'un gaz dans un solvant aqueux pendant une période de temps appropriée pour enrichir la teneur en oxygène dissous du solvant aqueux;
(ii) addition du composé complexe du platine (II) au solvant aqueux enrichi en oxygène de l'étape (i);
(iii) le mélange ou l'agitation du mélange de l'étape (ii) pour obtenir une solution claire;
(iv) stérilisation de la solution de l'étape (iii);
(v) le remplissage de la solution claire stérilisée de l'étape (iv) dans des contenants appropriés dans lesquels l'espace de tête n'est pas rempli avec un gaz;
(vi) rendre étanche les contenants tels que préparés à l'étape (v).

9. Procédé selon la revendication 8 pour la préparation d'une composition pharmaceutique, où ledit gaz est sélectionné parmi l'oxygène, des allotropes de l'oxygène et des combinaisons de ceux-ci.

10. Procédé selon la revendication 9 pour la préparation d'une composition pharmaceutique, où ledit bullage dudit gaz dans ledit solvant aqueux est exécuté durant une période de 30 minutes à 150 minutes.

11. Procédé selon la revendication 9 pour la préparation d'une composition pharmaceutique, où ledit bullage dudit gaz dans ledit solvant aqueux est exécuté de préférence durant une période de 90 minutes à 120 minutes.

12. Dispositif de préparation d'une composition pharmaceutique telle que revendiquée dans la revendication 1, dans lequel le procédé selon la revendication 8 est exécuté dans un ensemble ayant une taille de pore de surface d'aspersion de 0,2 µm à 7874 µm (0,31 pouce).

13. Utilisation d'une composition pharmaceutique selon la revendication 1 pour la fabrication d'un médicament pour le traitement d'une affection cancéreuse humaine ou animale.
